# EUROPEAN PATENT SPECIFICATION

(11) **EP 1 360 328 B1**
(45) Date of publication and mention of the grant of the patent: **05.09.2007**
(21) Application number: 02703838.9
(22) Date of filing: 07.02.2002
(51) Int. Cl.: C12Q 1/68

(54) **METHOD FOR THE ISOLATION OF EXTRACHROMOSOMAL DNA AND FOR THE USE OF SAID DNA FOR DIAGNOSING PATHOLOGIES**
VERFAHREN ZUR ISOLIERUNG VON EXTRACHROMOSOMALER DNA UND ZUR VERWENDUNG DIESER DNA ZUR ERKENNUNG VON KRANKHEITEN
METHODE D'ISOLEMENT DE L'ADN EXTRACHROMOSOMIQUE, ET METHODE DE DIAGNOSTIC BASEE SUR L'ANALYSE PAR ELECTROPHORESE DUDIT ADN

(30) Priority: 08.02.2001 IT SI20010002
(43) Date of publication of application: 12.11.2003
(73) Proprietor: Menesini, Maria Giulia, 53017 Radda in Chianti (SI) (IT)
(72) Inventor: Menesini, Maria Giulia, 53017 Radda in Chianti (SI) (IT)
(74) Representative: Raimondi, Adriana
(86) International application number: PCT/IT2002/000075
(87) International publication number: WO 2002/063040

(56) References cited:
- STROUN M ET AL: "ISOLATION AND CHARACTERIZATION OF DNA FROM THE PLASMA OF CANCER PATIENTS" EUROPEAN JOURNAL OF CANCER & CLINICAL ONCOLOGY, vol. 23, no. 6, 1987, pages 707-712, XP009002695 ISSN: 0277-5379
- STROUN M ET AL: "NEOPLASTIC CHARACTERISTICS OF THE DNA FOUND IN THE PLASMA OF CANCERPATIENTS" ONCOLOGY, S. KARGER AG, BASEL, CH, vol. 46, 1989, pages 318-322, XP000974054 ISSN: 0030-2414
- SALBE C ET AL: "Molecular detection of codon 12 K-RAS mutations in circulating DNA from serum of colorectal cancer patients." INTERNATIONAL JOURNAL OF BIOLOGICAL MARKERS, vol. 15, no. 4, October 2000 (2000-10), pages 300-307, XP009002683 ISSN: 0393-6155
- GREENSPOON S A ET AL: "QIAAMP SPIN COLUMNS AS A METHOD OF DNA ISOLATION FOR FORENSIC CASEWORK" JOURNAL OF FORENSIC SCIENCES, CALLAGHAN AND CO., CHICAGO, IL,, US, vol. 43, no. 5, September 1998 (1998-09), pages 1024-1030, XP000974645 ISSN: 0022-1198
- KRAMVIS A ET AL: "COMPARISON OF HEPATITIS B VIRUS DNA EXTRACTIONS FROM SERUM BY THE QIAAMP BLOOD KIT, GENERELEASER, AND THE PHENOL-CHLOROFORM METHOD" JOURNAL OF CLINICAL MICROBIOLOGY, WASHINGTON, DC, US, vol. 34, no. 11, 1 November 1996 (1996-11-01), pages 2731-2733, XP002054107 ISSN: 0095-1137

## Description

The present invention relates to a process for the extraction and isolation of extrachromosomal DNA at an adequate degree of purity, from a suitable biological sample withdrawn from a mammal and in particular from a human being. The invention also relates to an electrophoretic diagnostic method for detecting possible alterations of pathological consequence in the extrachromosomal DNA extracted and isolated by means of the process of the present invention, with a view to confirming or denying the suspect that the above mentioned mammal, or, in particular, the human being might be affected by a pathological form, whose presence could be diagnosed observing an alteration, inborn or induced, of its DNA, analysis that, in short, is known with the English expression ''DNA diagnosis".

For an in depth report on DNA diagnosis, see, for example, chapter 40, Medical Genetics, in "Current Medical Diagnosis and Treatment", 38th edition (1999), edited by Lawrence M. Tierney, Stephen J. Mc Phee and Maxine A, Papadakis and published by Appleton and Lange, publication which is incorporated by reference in the present description.

According to the present invention, by "suitable biologic sample" is meant a sample consisting of (a) whole blood or plasma/serum, or (b) the buffy coat fraction obtained by centrifuging heparinized blood or (c) the homogenate of tissues and cells. (It is known that by "buffy coat" is meant the layer consisting of leukocytes and platelets which forms between the layer of red blood cells and plasma by centrifuging whole blood).

It has long since been known that nucleic acids, in particular deoxyribonucleic acid (or DNA), constitute the key elements for the transmission of genetic information in all living organisms. DNA is comprised of a sequence of nitrogen bases: Adenine (A), Cytosine (C), Guanine (G) and Thymine (T). The sequences of nitrogen bases varying from a few units to millions of units in length, form the so-called genes whose functions have been identified and described in detail in the scientific literature. The research on genoma, the pool of all genes that characterize any specific species, aims at identifying the normal sequence of genes. Many genes have been isolated and identified, and various diseases have been characterized, brought about by their abnormalities. The research on the genoma, however, is still at a beginning stage because there are still hundred thousands of genes waiting for isolation and identification.

Circulating DNA study has recently prompted an increasing interest (see Anker et al, Cancer Metastasis Rev. 1999, 18:65-73). To know this DNA means to obtain viable means enabling early diagnosis of various diseases, for example, the onset of cancer, by non-invasive method.

Some genetic alterations have been identified and correlated with defined diseases using analytical methods such as Polymerase Chain Reaction (PCR), the Fluorescence in situ Hybridization (FISH), the in situ PCR (isPCR), the quantitative PCR, the sequence of circulating DNA and the RAS mutation and so on. For all of these analyses, it is necessary to have available a sample of isolated and purified circulating DNA/RNA, suitable for the object. It is well known that from a sample of blood plasma, serum, erythrocytes, leukocytes etc. can be obtained. The difficulty presented during the extraction of DNA from these fractions was mainly attributed to their high content of proteins and the possible contamination by hemoglobin which is considered harmful for the outcome of PCR analysis. To date various extraction methods are known but the DNA/RNA thus obtained often presents undefined or even unknown properties. Furthermore, these types of analyses, not only entail high cost and prolonged times, but are complicated and very often furnish uncertain results. The incidence is around 30-60 % only.

Therefore, it is necessary to provide a simple, reliable and rapid method to detect the DNA/RNA pattern in blood samples useful for the identification of DNA alterations and for the diagnosis of various related pathologies.

The essence of the present invention is based on the casual observation of a blood sample obtained from a calf affected by muscle dystrophy.

The extraction of circulating DNA with various methods shows that in a determined process DNA patterns with clear bands on agarose gel are obtained. It was observed that the DNA pattern of an autoimmune subject is different from that of an healthy individual. We postulated that the above mentioned observations should have more than a diagnostic value.

The main object of the present invention is to provide a process of extraction and isolation of extrachromosomal DNA instead of chromosomal DNA from a biological sample withdrawn from a mammal, in particular from a human being. The process should be rapid, simple and unexpensive and should also provide a warranty of obtaining isolated DNA of suitable purity, specially guaranteeing the absence of relevant amounts of contaminating substances such as the residual proteins and hemoglobin which could alter the outcome of electrophoretic analysis whereat the isolated DNA is submitted for diagnostic purposes.

A further object of the present invention is to provide a simple and reliable electrophoretic diagnostic method for detecting the eventual alterations of pathological significance in the DNA extracted and isolated, according to the above mentioned process.

According to the invention, the process of extraction and isolation of the extrachromosomal DNA comprises subjecting a biological sample withdrawn from a mammal and consisting of (a) whole blood or plasma/serum, or (b) a "buffy coat" fraction obtained by centrifuging heparinized blood, or (c) homogenized tissues or cells, to the following steps:
(1) adding a buffer to said biological sample;
(2) heating the resulting mixture to a temperature of about 25-100°C;
(3) centrifuging the product of step (2) and removing the supernatant;
(4) adding a detergent to the supernatant and maintaining the resulting mixture at about 25°C for 4-8 minutes;
(5) adding to the mixture of step (4) a salt selected from the halides of alkali metals or alkaline-earth metals and keeping the resulting mixture under stirring for about 8-12 minutes;
(6) centrifuging the mixture of step (5) and removing the extrachromosomal DNA-containing supernatant; and, optionally,
(7) concentrating said supernatant via known methods.

For the purposes of the invention it is obviously particularly relevant the case in which the above mentioned mammal is a human being, whose thus isolated extrachromosomal DNA is suitable for undergoing "DNA diagnosis".

There are various reagents which could be appropriately utilized as buffers [in step (1)] and detergents [in step (4)]. These buffers and detergents are well known to the experts in the techniques and methods used in the molecular biology laboratories.

A non-limiting example of buffer is the 2-amino-2-(hydroxymethyl)-1,3-propandiol, abbreviated as TBS, or tris buffer saline (pH =7.8).

A non-limiting example of detergent is sodium dodecylsulfate (SDS).

The salt in the step (5) is appropriately selected from the alkali metal chlorides and it is, preferably, potassium chloride.

The electrophoretic diagnostic method according to the invention for detecting possible alterations of pathological importance in the extrachromosomal DNA of a mammal suspected of being affected by a pathology (hereinbelow the "first mammal") comprises the steps consisting of:
(i) extracting and isolating by means of the above mentioned process of extraction and isolation from a biological sample withdrawn from the first mammal a quantity of extrachromosomal DNA sufficient for electrophoretically analyzing such DNA on agarose gel via known methods, thus obtaining a first electrophoretic map;
(ii) extracting and isolating by means of the above mentioned process of extraction and isolation from a biological sample withdrawn from a healthy mammal of the same species of the first mammal (hereinbelow the "second mammal") a quantity of extrachromosomal DNA sufficient for electrophoretically analyzing such DNA on agarose gel via known methods, thus obtaining a second electrophoretic map; and
(iii) comparing the first with the second electrophoretic map for detecting possible alterations of the extrachromosomal DNA of the first mammal with respect to that of the second mammal and evaluating the importance and the possible pathologic nature of such alterations.

To the ultimate aims of the invention, it is obviously particularly important the case wherein the first and the second mammal are human beings. Moreover, it is particularly useful that such human beings are in a parental relationship to each other.

The method is particularly even though non exclusively useful, for the accurate diagnosis of pathologies, possibly including also the onset stages of serious diseases, such as autoimmune or tumor diseases.

The present invention shall be now described in more details in the following examples for illustrative, non limiting purposes. Furthermore, variations and modifications can be made by the experts without thereby departing from the spirit and scope of the invention.

### Example 1

### Isolation of DNA from whole blood or plasma/serum

To 0.2-2 ml of whole blood or plasma/serum 0.1-2 volumes of buffer (preferably TBS, Tris buffer saline pH 7.8) were added. After heating to 25-100 °C (for example, by boiling for a few minutes), the sample was centrifuged at 13,000 rpm at 25°C for 5 minutes. The supernatant thus obtained was then treated with a detergent (preferably SDS; sodium dodecylsulfate, in TBS, at the final concentration of 2 %) for 5 minutes at 25°C. To this mixture KCl (from 0.1 to 6 M) was added and the resulting mixture, after agitation, was kept at 25°C for other 10 minutes. Following centrifugation at 13,000 rpm at room temperature for 10-20 minutes the supernatant thus obtained was ready for undergoing electrophoretic analysis on agarose gel (0.4-2 %). It is worthwhile noting that, if necessary, the sample could be subjected to further purification by means of the currently known methods, or the end product could be concentrated via well known procedures.

The end product is generally a liquid. The electrophoretic map, in the case of a sample of a healthy human being, analyzed on the agarose gel (0.4 %), shows bands having apparent molecular weight from 2 to 23 Kbs for plasma and serum and from 0.5 to 33 Kbs for whole blood.

### Example 2

### Isolation of DNA from nucleated blood cells or homogenized tissue

In order to isolate DNA from nucleated blood cells, heparinized blood (5-10 ml) was centrifuged at 3,000 rpm at room temperature for 3 minutes. The white layer, between plasma and red cells, hereinbelow the "Buffy Coat" (BC), was isolated with care, avoiding any possible contamination with either plasma or red cells. The BC thus obtained was suitable for undergoing the treatment described in Example 1.

For tissues or tumor cells in vitro a quantity of material was homogenized in an equivalent volume of buffer (preferably TBS) for about 5-10 minutes and was thus suitable for undergoing the treatment described in Example 1:

Under normal conditions the end product isolated from nucleated blood cells, is a clear and odorless liquid. The DNA pattern analyzed (for example from a human sample) by electrophoresis on 0.4 % agarose gel shows bands from 0.5 to 33 Kbs for the nucleated blood cells; for other types of cells the DNA pattern varies according to the sample under study.

As just described above, the basic concept of the present invention consists in identifying and isolating extrachromosomal DNA. The rate at which it has been possible to isolate the DNA present in the samples under study supports the validity and reliability of the process.

Using the process of the invention it was possible, for example, to observe that the DNA pattern extracted from the BC of a healthy calf is very different from that extracted from the BC of a calf affected by muscular dystrophy. Similar results were also obtained from human samples, for example in the case of a patient who suffered from an autoimmune disease. Relevant data show that the process of present invention allows the DNA pattern alterations linked to a determined disease be detected through the modifications of its electrophoretic properties and also of its chimico-physical ones. This clearly indicates that the present invention provides a useful tool to clinical researchers for the mutated genes identification and for the diagnosis of the diseases caused by such mutations.

## Claims

1. A process for the extraction and isolation of extrachromosomal DNA from a biological sample withdrawn from a mammal consisting of (a) whole blood or plasma/serum, or (b) the buffy coat fraction obtained by centrifuging heparinized blood or (c) the homogenate of tissues and cells, which comprises the steps of:
(1) adding a buffer to said biological sample;
(2) heating the resulting mixture to a temperature of about 25-100°C;
(3) centrifuging the product of step (2) and removing the supernatant;
(4) adding a detergent to the supernatant and maintaining the resulting mixture at about 25°C for 4-8 minutes;
(5) adding to the mixture of step (4) a salt selected from the halides of alkali metals or alkaline-earth metals and keeping the resulting mixture under stirring for about 8-12 minutes;
(6) centrifuging the mixture of step (5) and removing the extrachromosomal DNA-containing supernatant; and, optionally,
(7) concentrating said supernatant via known methods.

2. The process of claim 1, wherein the biological sample is withdrawn from a human being.

3. The process of claim 1, wherein the buffer is TBS, tris buffer saline (pH=7.8).

4. The process of claim 1, wherein the detergent is sodium dodecylsulfate.

5. The process of claim 1, wherein the salt of step (5) is selected from the alkali-metal chlorides and is preferably potassium chloride.

6. An electrophoretic diagnostic method for detecting possible alterations of pathological importance in the extrachromosomal DNA of a mammal suspected of being affected by a pathology (hereinbelow the "first mammal") which comprises the steps of:
(i) extracting and isolating by means of the process of claim 1 from a biological sample withdrawn from said first mammal a quantity of extrachromosomal DNA sufficient for electrophoretically analyzing such DNA, on agarose gel via known methods, obtaining a first electrophoretic map;
(ii) extracting and isolating by means of the process of claim 1 from a biological sample withdrawn from a healthy mammal of the same species of the first mammal (hereinbelow the "second mammal") a quantity of extrachromosomal DNA sufficient for electrophoretically analyzing such DNA on agarose gel via known methods, obtaining a second electrophoretic map; and
(iii) comparing the first with the second electrophoretic map for detecting possible alterations of the extrachromosomal DNA of the first mammal with respect to that of the second mammal and evaluating the importance and the possible pathologic nature of such alterations.

7. The method of claim 6, wherein said first and second mammal are human beings.

8. The method of claim 7, wherein said human beings are in parental relationship to each other.

9. The method of claim 6, wherein the pathology is an autoimmune or tumor disease.

10. Use of a sample of extrachromosomal DNA, as a reference standard in the method of claims 6 to 9.

## Patentansprüche

1. Verfahren für die Extraktion und Isolation extrachromosomaler DNA aus einer biologischen Probe, die von einem Säuger genommen wurde, bestehend aus (a) Gesamtblut oder Plasma/Serum oder (b) der Buffycoatfraktion, erhalten durch Zentrifugation von mit Heparin behandeltem Blut oder (c) dem Homogenat von Geweben und zellen, das die folgenden Schritte umfaßt:
(1) Zugabe eines Puffers zu der biologischen Probe;
(2) Erwärmung der resultierenden Mischung auf eine Temperatur von ungefähr 25 bis 100°C;
(3) zentrifugation des Produkts aus Schritt (2) und Entfernung des Überstands;
(4) Zugabe eines Detergens zu dem Überstand und Erhalt der resultierenden Mischung bei ungefähr 25°C für 4 bis 8 Minuten;
(5) Zugabe eines Salzes, ausgewählt aus den Halogeniden von Alkalimetallen oder Erdalkalimetallen zu der Mischung aus Schritt (4) und Halten der resultierenden Mischung unter Rühren für ungefähr 8 bis 12 Minuten;
(6) zentrifugation der Mischung aus Schritt (5) und Entfernung von extrachromosomaler DNA-haltigem Überstand; und optional
(7) Konzentration des Überstands durch bekannte Verfahren.

2. Verfahren gemäß Anspruch 1, wobei die biologische Probe von einem Menschen entnommen wurde.

3. Verfahren gemäß Anspruch 1, wobei der Puffer TBS, Trispuffersalzlösung (pH = 7,8) ist.

4. Verfahren gemäß Anspruch 1, wobei das Detergens Natriumdodecylsulfat ist.

5. Verfahren gemäß Anspruch 1, wobei das Salz aus Schritt (5) aus Alkalimetallchloriden ausgewählt ist und vorzugsweise Kaliumchlorid ist.

6. Elektrophoretisches Diagnoseverfahren zum Nachweis möglicher Veränderungen pathologischer Wichtigkeit in der extrachromosomalen DNA eines Säugers, von dem vermutet wird, daß er von einer Pathologie betroffen ist (hiernach der "erste Säuger"), das die Schritte umfaßt:
(i) Extraktion und Isolation durch das Verfahren gemäß Anspruch 1 einer Menge an extrachromosomaler DNA, die für eine elektrophoretische Analyse der DNA auf einem Agarosegel genügt, über bekannte Verfahren aus einer biologischen Probe, die von dem ersten Säuger genommen wurde, Erhalt einer ersten elektrophoretischen Karte;
(ii) Extraktion und Isolation durch das Verfahren gemäß Anspruch 1 einer Menge an extrachromosomaler DNA, die für eine elektrophoretische Analyse der DNA auf einem Agarosegel genügt, über bekannte Verfahren aus einer biologischen Probe, die von einem gesunden Säuger derselben Art wie dem ersten Säuger (hiernach der "zweite Säuger") genommen wurde, Erhalt einer zweiten elektrophoretischen Karte; und
(iii) Vergleich der ersten mit der zweiten elektrophoretischen Karte zum Nachweis möglicher Veränderungen der extrachromosomalen DNA des ersten Säugers in Hinblick auf diejenige des zweiten Säugers, und Bewertung der Wichtigkeit und der möglichen pathologischen Natur solcher Veränderungen.

7. Verfahren gemäß Anspruch 6, wobei der erste und zweite Säuger Menschen sind.

8. Verfahren gemäß Anspruch 7, wobei die Menschen sich in verwandtschaftlicher Beziehung zueinander befinden.

9. Verfahren gemäß Anspruch 6, wobei die Pathologie eine Autoimmun- oder Tumorerkrankung ist.

10. Verwendung einer Probe extrachromosomaler DNA als Referenzstandard in dem Verfahren gemäß den Ansprüchen 6 bis 9.

## Revendications

1. Procédé pour l'extraction et l'isolement d'ADN extrachromosomique à partir d'un échantillon biologique extrait d'un mammifère consistant en (a) du sang complet ou du plasma/sérum ou (b) la fraction de couche leucocytoplaquettaire obtenue par centrifugation de sang hépariné ou (c) le broyat de tissus et cellules, qui comprend les étapes de :
(1) addition d'un tampon au dit échantillon biologique ;
(2) chauffage du mélange résultant à une température d'environ 25-100 °C ;
(3) centrifugation du produit de l'étape (2) et retrait du surnageant ;
(4) addition d'un détergent au surnageant et maintien du mélange résultant à environ 25°C pendant 4-8 minutes ;
(5) addition au mélange de l'étape (4) d'un sel choisi parmi les halogénures de métaux alcalins ou de métaux alcalino-terreux et maintien du mélange résultant sous agitation pendant environ 8-12 minutes ;
(6) centrifugation du mélange de l'étape (5) et retrait du surnageant contenant l'ADN extrachromosomique ; et, éventuellement,
(7) concentration dudit surnageant par des méthodes connues.

2. Procédé de la revendication 1, dans lequel l'échantillon biologique est extrait d'un être humain.

3. Procédé de la revendication 1, dans lequel le tampon est le TBS, tampon Tris-salin (pH = 7,8).

4. Procédé de la revendication 1, dans lequel le détergent est le dodécylsulfate de sodium.

5. Procédé de la revendication 1, dans lequel le sel de l'étape (5) est choisi parmi les chlorures de métaux alcalins et est préférentiellement du chlorure de potassium.

6. Méthode de diagnostic électrophorétique pour détecter des altérations possibles d'importance pathologique dans l'ADN extrachromosomique d'un mammifère suspecté d'être affecté par une pathologie (ci-dessous le « premier mammifère ») qui comprend les étapes de :
(i) extraction et isolement au moyen du procédé de la revendication 1 à partir d'un échantillon biologique extrait dudit premier mammifère d'une quantité d'ADN extrachromosomique suffisante pour analyser électrophorétiquement un tel ADN sur gel d'agarose par des méthodes connues, obtenant une première carte électrophorétique ;
(ii) extraction et isolement au moyen du procédé de la revendication 1 à partir d'un échantillon biologique extrait d'un mammifère sain de la même espèce que le premier mammifère (ci-dessous le « second mammifère ») d'une quantité d'ADN extrachromosomique suffisante pour analyser électrophorétiquement un tel ADN sur gel d'agarose par des méthodes connues, obtenant une seconde carte électrophorétique ; et
(iii) comparaison de la première carte électrophorétique avec la seconde pour détecter de possibles altérations de l'ADN extrachromosomique du premier mammifère par rapport à celui du second mammifère et évaluation de l'importance et de la nature pathologique possible de telles altérations.

7. Méthode de la revendication 6, dans laquelle les dits premier et second mammifères sont des êtres humains.

8. Méthode de la revendication 7, dans laquelle les dits êtres humains ont des relations de parenté.

9. Méthode de la revendication 6, dans laquelle la pathologie est une maladie auto-immune ou tumorale.

10. Utilisation d'un échantillon d'ADN extrachromosomique, comme référence standard dans la méthode des revendications 6 à 9.
